# EUROPÄISCHE PATENTSCHRIFT

(11) **EP 0 732 097 B1**
(45) Veröffentlichungstag und Bekanntmachung des Hinweises auf die Patenterteilung: **25.11.1998**
(21) Anmeldenummer: 96103778.5
(22) Anmeldetag: 11.03.1996
(51) Int. Cl.: A61J 1/16

(54) **Blutplasmahalterung für Plasmabeutel während des Einfrierens**
Support for plasma pouch during freezing
Support pour poche de plasma pendant la congélation

(30) Priorität: 15.03.1995 DE 29504401 U
(43) Veröffentlichungstag der Anmeldung: 18.09.1996
(73) Patentinhaber: Heraeus Instruments GmbH & Co. KG, 63450 Hanau (DE)
(72) Erfinder: Stetter, Thomas, 73557 Mutlangen (DE)
(74) Vertreter: Kühn, Hans-Christian

(56) Entgegenhaltungen:
- EP-A- 0 190 396
- FR-A- 893 719
- GB-A- 1 153 288
- US-A- 4 194 369

## Beschreibung

Die Erfindung bezieht sich auf eine Blutplasmahalterung für Plasmabeutel während des Einfrierens.

Blutplasma wird für längerfristige Lagerung in Plastiktüten gefüllt, diese werden verschlossen, im allgemeinen verschweißt, es entstehen wabbelige Kissen. Diese werden eingefroren und bei tiefer Temperatur gelagert. Das Einfrieren findet typischerweise bei Temperaturen zwischen -40° und -70°C statt. Entscheidend ist dabei die Einfrierzeit. Die Kerntemperatur des Plasmas innerhalb des Beutels soll innerhalb einer Stunde -23°C erreichen.

Nach dem Stand der Technik werden drei unterschiedliche Verfahren für das Einfrieren von in Beuteln abgefülltem Blutplasma eingesetzt. Nach dem ersten Verfahren erfolgt das Einfrieren im Tiefkältebad. Damit kann eine recht rasche Abkühlung erreicht werden. Anzustreben ist allerdings, daß ein direkter Kontakt zwischen Plasmabeutel und Badflüssigkeit vermieden wird. Dadurch werden flüssigkeitsdichte Ummantelungen erforderlich, diese bedeuten zusätzlichen Aufwand, insbesondere praktische Schwierigkeiten. Nachteilig beim Tiefkältebad ist auch, daß schädliche Dämpfe von der Flüssigkeit aufsteigen. Diese stellen eine Belastung für das Personal dar.

Das Einfrieren von Plasma nach dem zweiten Verfahren, nämlich im Tiefkälteschrank mit statischer Luftverteilung, führt nicht mit Sicherheit zu der benötigten raschen Abkühlung. Beim dritten Verfahren, nämlich dem Einfrieren im Umluftverfahren, ist eine ausreichend rasche Abkühlung möglich; weiterhin werden Dämpfe von Badflüssigkeiten vermieden. Nach dem Stand der Technik werden die Plasmabeutel in Klappschieber eingelegt, die Klappschieber werden senkrecht in den kalten Luftstrom gestellt. Für die unterschiedlichen Beutelabmessungen - gängig sind derzeit Beutel mit einem Inhalt von 300, 500, 700 und 1000 ml - müssen unterschiedliche Klappschieber eingesetzt und damit auch vorrätig gehalten werden. Dies ist nachteilig.

Hier setzt nun die Erfindung ein. Sie hat es sich zur Aufgabe gemacht, eine Blutplasmahalterung für Plasmabeutel während des Einfrierens im Umluftverfahren anzugeben, die universell einsetzbar ist, einen raschen Wärmeübergang ermöglicht, eine einfache Entnahme der gefrorenen Plasmabeutel zuläßt und bequem handzuhaben ist.

Diese Aufgabe wird ausgehend von der Blutplasmahalterung der eingangs genannten Art, gelöst durch ein metallisches Stapelgestell
- mit a) einem Grundgestell, das zwei seitliche Rahmenteile und mindestens drei zwischen diesen angeordnete und mit ihnen verbundene, zueinander parallel und im Abstand voneinander angeordnete Lochbleche aufweist,
- mit b) Druckplatten, die am zugehörigen Lochblech beweglich gehalten, aus einem gelochten Blech gefertigt, oberhalb des zugehörigen Lochblechs angeordnet sind und von denen jeweils eine pro Lochblech vorgesehen ist,
- mit c) einer Federvorrichtung, die zwischen dem Grundgestell und den Druckplatten angeordnet ist und mehrere Federn (z. B. Blattfedern) aufweist, die die Druckplatten elastisch gegen das jeweils zugehörige Lochblech vorbelasten.

Erfindungsgemäß werden die Plasmabeutel nicht mehr in senkrechtem Zustand, sondern waagerecht liegend eingefroren. Das erfindungsgemäße Stapelgestell ist so groß ausgeführt, daß alle herkömmlichen Plasmabeutel unabhängig von Größe, Füllmenge und Verwendung eines Umkartons, im Stapelgestell aufgenommen werden können. Dies wird durch die variable Klemmung erreicht, die die Druckplatten zusammen mit der Federvorrichtung auf die Plasmabeutel ausüben. Durch diese variable Klemmung werden unterschiedliche Beutelgrößen und Füllmengen ausgeglichen. Durch den Druck der Druckplatten wird eine ungleichmäßige Ausdehnung des Plasmas beim Einfrieren vermieden. Auf diese Weise wird ein gleichmäßig dicker, tiefgefrorener Plasmabeutel erhalten, der dann in einem Lagerschrank, beispielsweise Tiefkühlschrank, über eine längere Zeit aufbewahrt werden kann.

In dem erfindungsgemäßen Stapelgestell können mehrere Plasmabeutel, typischerweise drei pro Stapelgestell, eingefroren werden. Die Lochbleche und die Druckplatten sind so ausgebildet, daß zwischen Ihnen ein Plasmabeutel mit 1000 ml Inhalt untergebracht werden kann. An seiner Stelle können aber auch z. B. zwei Plasmabeutel mit je 300 ml angeordnet werden. Die Handhabung bei der Bestückung und bei der Entnahme ist sehr einfach. Es ist möglich, Umkartons beim Einfrieren einzusetzen.

Durch die Federvorrichtung werden die Druckplatten so gegenüber dem zugehörigen Lochblech elastisch vorbelastet, daß ein ausreichender Andruck eines dazwischen angeordneten Plasmabeutels erfolgt, andererseits aber per Hand die Druckplatten angehoben werden können, um einen Plasmabeutel zu entnehmen bzw. einzulegen. Durch die Verwendung gelochter Bleche wird ein verbesserter Wärmeaustausch erreicht; die gekühlte Umluft kann unmittelbar mit dem Beutel des Plasmabeutels in Kontakt kommen.

In einer verbesserten Weiterbildung sind an den Druckplatten oder am Grundgestell, dann insbesondere an den Lochblechen, Abstandshalter vorgesehen, die in Gegenrichtung zur Federkraft auf die Druckplatte wirken und einen minimalen, vertikalen Abstand jeder Druckplatte vom zugehörigen Lochblech festlegen. Dieser minimale Abstand, der durch die Abstandshalter vorgegeben ist, ist kleiner als die Dicke des kleinsten, einzufrierenden Beutels. Durch die Abstandshalter bleibt stets ein minimaler Abstand zwischen den Lochblechen und der zugehörigen Druckplatte erhalten, dies vereinfacht das Öffnen. Weiterhin wird der Weg der Druckfedern limitiert.

In einer weiteren, bevorzugten Ausführung sind an den Druckplatten und/oder an den Lochblechen parallel zu den Rahmenteilen verlaufende Seitenränder vorgesehen, die vorzugsweise einstückig mit der Druckplatte bzw. dem Lochblech verbunden sind. In einer günstigen Weiterbildung sind sie durch Umbiegen des Zuschnitts für die Druckplatte bzw. das Lochblech erstellt. Durch sie wird die seitliche Ausdehnung vorgegeben bzw. begrenzt. Sie können die Funktion der Abstandshalter übernehmen. Sind sowohl an den Druckplatten als auch am zugehörigen Lochblech jeweils derartige Seitenränder vorgesehen, so ist der Innenraum zwischen den Druckplatten und dem zugehörigen Lochblech seitlich abgeschlossen, unabhängig vom vertikalen Abstand zwischen beiden.

In einer bevorzugten Ausführung hat die Federvorrichtung pro Druckplatte jeweils zwei seitliche Blattfedern, die eine Länge haben, die zumindest 50 % der Tiefenabmessung der Druckplatte entspricht. Diese Blattfedern liegen seitlich im spitzen Winkel an den Druckplatten an. Zwischen diesen Federn klemmen die Druckplatten. Aufgrund des spitzen Anlagewinkels ist nur ein kleiner Federweg nötig, um die Federung über den gesamten Abstandsbereich zwischen Lochblech und Druckplatte aufrecht zu erhalten. Durch die beschriebene Ausbildung der Federn wird erreicht, daß die Druckplatte einseitig, beispielsweise von vorn, hochgeklappt werden kann, als wäre sie anscharniert.

In einer bevorzugten Ausführung wird jede Druckplatte jeweils nur durch ihr eigenes Gewicht und die Federvorrichtung im Grundgestell gehalten. Das Gewicht der Druckplatte allein reicht nicht aus, um eine ungleichmäßige Ausdehnung während des Einfrierens zu vermeiden. Dies wird im wesentlichen durch die Kraft der Federvorrichtung erreicht. Darüberhinaus ist die Druckplatte nicht in irgendeiner Form mit dem Grundgestell verbunden. Die Ausbildung der Federvorrichtung selbst ist beliebig, es können unterschiedliche Federn eingesetzt werden, neben den beschriebenen Blattfedern auch Schenkelfedern, Zugfedern, Druckfedern usw.

Vorzugsweise wird jede Druckplatte an mindestens vier Punkten, nämlich zwei linksseitigen und zwei rechtsseitigen Punkten durch die Federvorrichtung gegen das zugehörige Lochblech vorbelastet. Günstig ist dabei, daß diese Angriffspunkte einen großen Abstand voneinander haben, weiterhin sollen sie symmetrisch zur Tiefenmitte der Druckplatte angreifen.

Schließlich hat es sich als besonders günstig herausgestellt, die beiden seitlichen Rahmenteile so offen wie möglich auszubilden, insbesondere aus einem Stabmaterial, beispielsweise einem dünnen Rundmaterial, durch Biegen herzustellen. Sie haben dann die Form eines schmalen, im wesentlichen rechteckförmigen Rahmens. Eine Stapelbarkeit wird beispielsweise dadurch erreicht, daß die seitlichen Rahmenteile am oberen Ende nach innen abgebogen sind, sodaß der untere Rand eines darüber befindlichen Stapelgestells auf dem Bereich der Innenabbiegung aufliegen kann.

Weitere Vorteile und Besonderheiten der Erfindung ergeben sich aus den übrigen Ansprüchen sowie der nun folgenden Beschreibung eines nicht einschränkend zu verstehenden Ausführungsbeispiels der Erfindung, das unter Bezugnahme auf die Zeichnung näher erläutert wird. In dieser zeigen:
FIG. 1 eine Seitenansicht der Blutplasmahalterung nach der Erfindung mit drei Lochblechen, zugehörigen Druckplatten und einer insgesamt sechs Blattfedern aufweisenden Federvorrichtung,
FIG. 2 ein Schnittbild entlang der Schnittlinie II-II in FIG. 1 und
FIG. 3 eine Draufsicht auf die Blutplasmahalterung gem. FIG. 1.

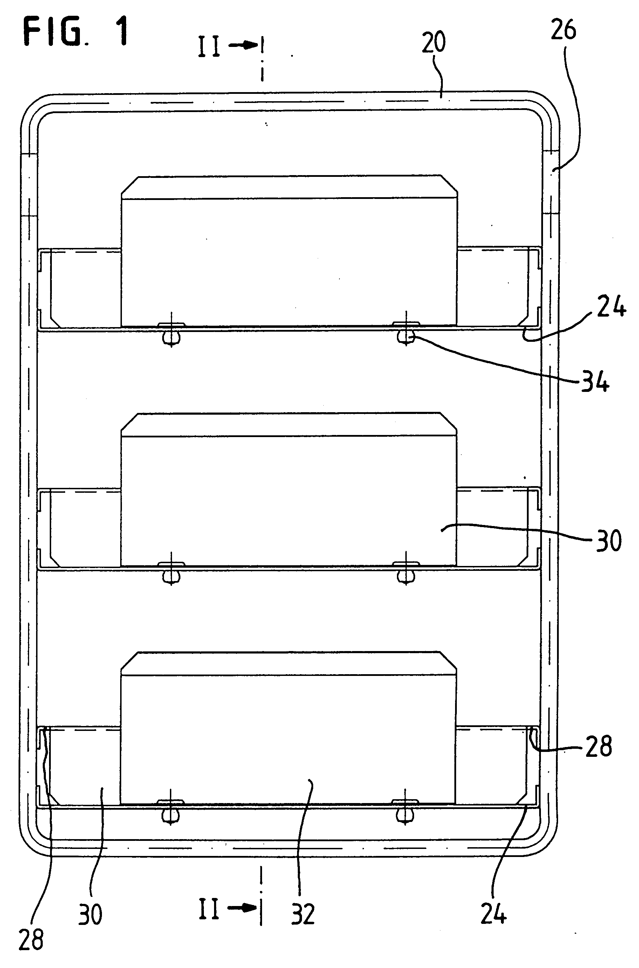

Das erfindungsgemäße Stapelgestell für Plasmaeinfrierung in Beuteln ist aus Edelstahl gefertigt. Es hat erstens ein Grundgestell, das aus zwei seitlichen Rahmenteilen 20, 22 und drei zwischen diesen angeordneten und mit ihnen verbundenen Lochblechen zusammengesetzt ist. In einer bevorzugten Ausführung ist es als Schweißkonstruktion ausgeführt. Die beiden Rahmenteile sind aus Rundmaterial, beispielsweise 5 mm Durchmesser, gebogen, sie sind als Rahmen ausgeführt, der, wie insbesondere aus FIG. 1 ersichtlich ist, ein Rechteck mit gerundeten Enden ist. Er hat in einer typischen Ausführung die Höhe 22,5 cm und die Tiefe 16 cm. Im oberen Bereich sind die beiden Rahmenteile 20, 22 leicht nach innen abgewinkelt, es liegt eine Abschrägung 26 vor. Sie bewirkt einen Versatz von etwa 10 mm nach innen hin. Auf der Abschrägung können die unteren Teilstücke eines darüber angeordneten Stapelgestells aufliegen. Die oberen Teilstücke jedes Rahmenteils 22 dienen gleichzeitig als Handhabe (Handgriffe).

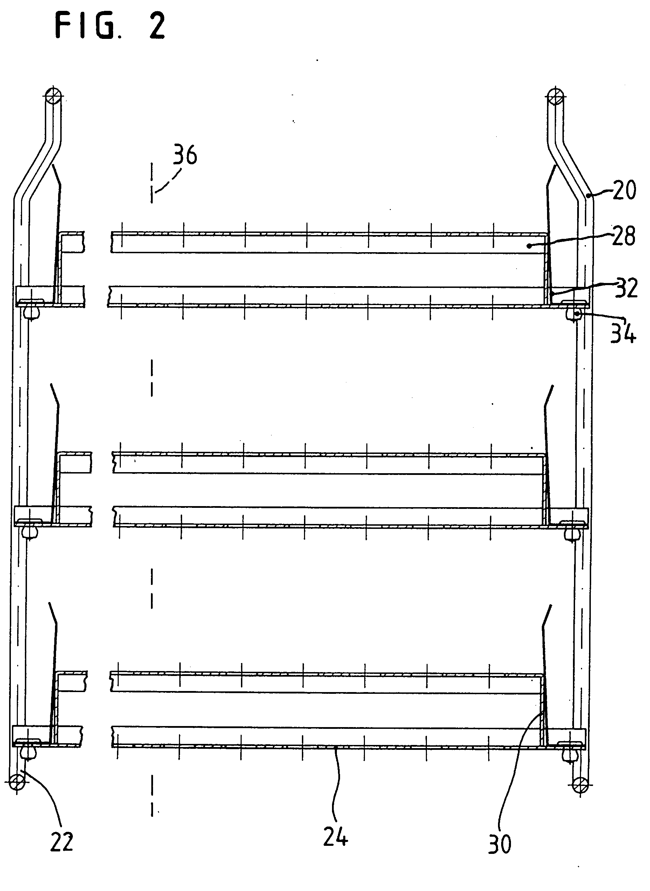

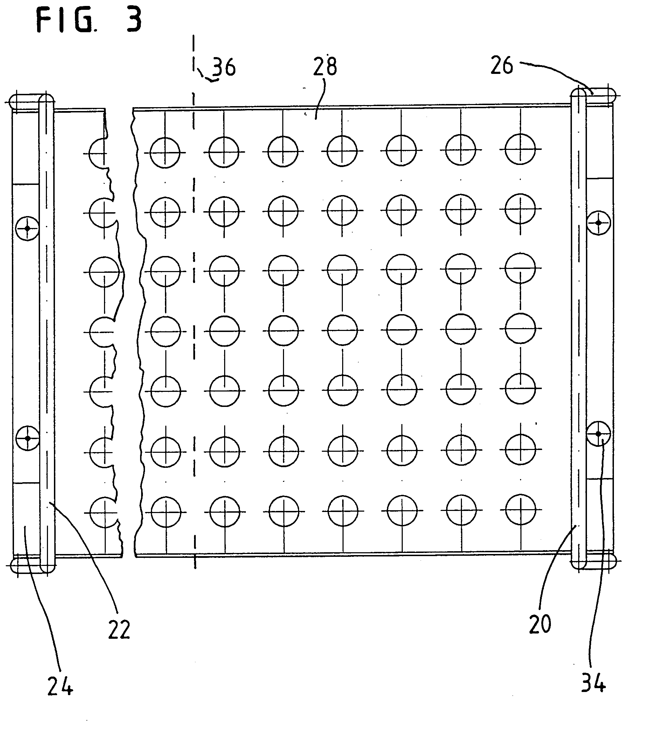

Ähnlich Regalbrettern sind zwischen den beiden Rahmenteilen 20, 22 in gleichem Abstand voneinander insgesamt drei Lochbleche 24 angeordnet. Sie liegen am Innenrand der vertikalen Teile der Rahmenteile 20, 22 an und sind dort befestigt, insbesondere angeschweißt. Die Lochbleche 24 haben vorn und hinten, wie FIG. 2 zeigt, einen hochstehenden, abgewinkelten Rand, dieser steht etwa 5 - 7 mm hoch. Die Lochbleche 24 sind aus dünnem Blech gefertigt, beispielsweise Blechstärke 0,6 - 1 mm. Ihre Lochung ist aus FIG. 3 ersichtlich.

Jedem Lochblech 24 ist eine Druckplatte 28 zugeordnet. Die Druckplatten 28 haben die gleiche Tiefe wie die Lochbleche 26, sie haben ebenfalls vorn und hinten den bereits für die Lochbleche 24 beschriebenen Rand, nur steht dieser diesmal nach unten, nicht wie bei den Lochblechen 24 nach oben. Anders ausgedrückt weisen die Ränder aufeinander zu, FIG. 2 zeigt dies. In ihrer Breite sind die Druckplatten 28 etwas kleiner als die Lochbleche 24, sie haben eine Breite, die ungefähr dem lichten Abstand zwischen den beiden oberen Handgriffen der Rahmenteile 20, 22 entspricht. Seitlich haben die Druckplatten 28 Seitenränder 30, die etwa 22 mm hoch sind. Bei nicht gefülltem Stapelgestell liegen sie, schon aufgrund der Schwerkraft, unten auf dem zugehörigen Lochblech 24 auf. Auf diese Weise wird der geringste Abstand zwischen Lochblech 24 und zugehöriger Druckplatte 28 vorgegeben. Die Druckplatten 28 sind ebenfalls aus einem Blechmaterial, vorzugsweise aus demselben Material wie die Lochbleche 24, gefertigt. Sie haben eine mit den Lochblechen übereinstimmende Lochung, wie insbesondere FIG. 2 zeigt, es fluchten sogar (zufällig) die Löcher übereinandergeordneter Lochbleche 24 und Druckplatten 28 in der korrekten Montageposition.

Außer durch die Schwerkraft liegen die Druckplatten 28 insbesondere durch eine Federvorrichtung gegen die zugehörigen Lochbleche 24 an, auf die Federvorrichtung wird im folgenden im einzelnen eingegangen. Wie die Figuren 1 und 2 zeigen, sind im Ausführungsbeispiel insgesamt sechs Blattfedern 32 vorgesehen, die L-förmig ausgeführt sind. Mit einem waagerechten, kurzen L-Schenkel sind sie am gegenüber der Druckplatte 28 überstehenden Seitenbereich des Lochblechs 24 fixiert, die Verbindung erfolgt im gezeigten Beispiel durch zwei Blindniete 34 pro Blattfeder 32. Ihr längerer Schenkel steht im wesentlichen vertikal, er liegt in einem sehr spitzen Winkel an der die Druckplatte 28 und den Seitenrand 30 verbindenden Kante an. Auf diese Weise ist die Federbewegung zwischen der in FIG. 2 gezeigten tiefsten Position jeder Druckplatte 28 und ihrem größtmöglichen Abstand von dem Lochblech 24, der etwa 17 mm höher liegt, ausgesprochen gering. Die Blattfedern 32 haben an ihrem oberen, freien Ende eine nach außen weisende Abschrägung, die ein Einsetzen einer entnommenen Druckplatte 28 von oben vereinfacht. Wie FIG. 1 zeigt, sind die Blattfedern 32 relativ lang, sie erstrecken sich über eine Tiefe von etwa 10 cm, also etwa 2/3 der Tiefe des Lochblechs 24 bzw. der Druckplatte 28. Damit bewirken sie über eine relativ große Tiefenabmessung einen Druck auf die zugehörige Druckplatte 28. Die Druckplatte 28 kann dadurch vorn (links oder rechts in FIG. 1) hochgehoben werden, sie bleibt dann hinten tief, anders ausgedrückt verhält sie sich wie ein anscharniertes Teil. Sie kann aber auch gänzlich nach oben entnommen werden. Hierzu ist oberhalb jeder Etage ausreichend Platz. Sie kann aber auch nach vorn bzw. hinten herausgezogen werden, wenn ihre Seitenränder 30 über den vertikal nach oben vorstehenden Rahmen des Lochblechs 24 gehoben werden.

Die Blattfedern 32 halten die Druckplatte 28 zwischen sich. Aufgrund der sehr steilen Anordnung der Blattfedern 32 haben die Druckplatten 28 nur ein sehr geringes seitliches Spiel, das im Bereich weniger Zehntel-Millimeter liegt. Die Blattfedern ermöglichen einen sehr einfachen Zusammenbau der Anordnung, da sie auch im freien (ausgefederten) Zustand der Federn 32 den Weg für das Einschieben einer Druckplatte 28 nicht blockieren oder behindern. Sie sind dadurch eine besonders günstige, konstruktive Ausbildung der Federvorrichtung. Andere Ausbildungen der Federvorrichtung sind dadurch allerdings nicht ausgeschlossen. So können Schenkelfedern benutzt werden, die bei Entnahme der Druckplatte 28 an Abstützungen anliegen, weiterhin haben die Druckplatten 28 Anlaufschrägen. Auf diese Weise ist ein Einschieben einer Druckplatte 28 möglich, ohne die Schenkelfedern separat von ihren Abstützungen anheben zu müssen. Die Schenkelfedern liegen mit quer zu den Rahmenteilen 20, 22 verlaufenden freien Endbereichen auf der Oberfläche der Druckplatte 28 auf. Ihr anderer Schenkel ist am Lochblech 24 gehalten.

Mit 36 ist in den Figuren 2 und 3 eine Klappsymmetrieebene bezeichnet. Zu dieser Ebene ist die rechte, vollständig gezeichnete Hälfte spiegelbildlich zur linken, nur teilweise dargestellten Hälfte.

## Patentansprüche

1. Blutplasmahalterung für Plasmabeutel während des Einfrierens, gekennzeichnet durch ein metallisches Stapelgestell
- mit a) einem Grundgestell, das zwei seitliche Rahmenteile (20, 22) und mindestens drei zwischen diesen angeordnete und mit ihnen verbundene, zueinander parallel und im Abstand voneinander angeordnete Lochbleche (24) aufweist,
- mit b) Druckplatten (28), von denen jeweils eine pro Lochblech (24) vorgesehen ist, die am zugehörigen Lochblech (24) beweglich gehalten, aus einem gelochten Blech gefertigt und oberhalb des zugehörigen Lochblechs (24) angeordnet sind und
- mit c) einer Federvorrichtung, die zwischen dem Grundgestell und den Druckplatten (28) angeordnet ist und mehrere Federn (Blattfeder 32) aufweist, die die Druckplatten (28) elastisch gegen das jeweils zugehörige Lochblech (24) vorbelasten.

2. Blutplasmahalterung nach Anspruch 1, dadurch gekennzeichnet, daß die Druckplatten (28) oder das Grundgestell, insbesondere die Lochbleche (24), Abstandshalter aufweisen, die in Gegenrichtung zum vertikal auf die Druckplatte (28) einwirkenden Federkraft Druck vorspringen und einen minimalen, vertikalen Abstand jeder Druckplatte (28) vom zugehörigen Lochblech (24) festlegen.

3. Blutplasmahalterung nach Anspruch 1, dadurch gekennzeichnet, daß an den Druckplatten (28) und/oder an den Lochblechen (24) parallel zu den Rahmenteilen (20, 22) verlaufende Seitenränder (30) vorgesehen sind, die vorzugsweise einstückig mit der Druckplatte (28) bzw. dem Lochblech (24) verbunden sind.

4. Blutplasmahalterung nach Anspruch 1, dadurch gekennzeichnet, daß die Fläche einer Druckplatte (28) sich um maximal 20 % von der Fläche des zugehörigen Lochblechs (24) unterscheidet.

5. Blutplasmahalterung nach Anspruch 1, dadurch gekennzeichnet, daß die Federvorrichtung pro Druckplatte (28) jeweils zwei seitliche Blattfedern (32) aufweist, die eine Länge haben, die zumindest 50 % der Tiefenabmessung der Druckplatte (28) entspricht.

6. Blutplasmahalterung nach Anspruch 1, dadurch gekennzeichnet, daß an jeder Druckplatte (28) die Federn (Blattfeder 32) der Federvorrichtung nur seitlich und vorzugsweise linienhaft anliegen, so daß die Druckplatte (28) nach oben aus den sie haltenden Federn herausgehoben werden kann.

7. Blutplasmahalterung nach Anspruch 1, dadurch gekennzeichnet, daß pro Druckplatte (28) jeweils zwei seitliche, L-förmig ausgebildete Blattfedern (32) vorgesehen, die einen kurzen L-Schenkel aufweisen, der am Lochblech (24) befestigt ist.

8. Blutplasmahalterung nach Anspruch 1, dadurch gekennzeichnet, daß die Lochbleche (24) und die Druckplatten (28) Löcher aufweisen, die mindestens 20 % der Fläche des jeweiligen Teils ausmachen.

9. Blutplasmahalterung nach Anspruch 1, dadurch gekennzeichnet, daß die seitlichen Rahmenteile (20, 22) aus einem Stangenmaterial, insbesondere Rundmaterial gebogen und im wesentlichen rechteckförmig ausgeführt sind.

10. Blutplasmahalterung nach Anspruch 1, dadurch gekennzeichnet, daß die Rahmenteile (20, 22) an ihren oberen, freien Enden nach innen im Bereich einer Abschrägung (26) abgebogen sind, wodurch eine Auflage für eine darüber gestapelte Blutplasmahalterung erreicht wird.

## Claims

1. A blood plasma holder for plasma bags during freezing, characterised by a metallic stacking rack
- with a) a base rack which has two lateral frame parts (20, 22) and at least three perforated sheets (24) arranged therebetween and connected therewith, arranged parallel to each other and spaced from each other.
- with b) pressure plates (28), one of which is provided in each case per perforated sheet (24), which are held so as to be movable on the associated perforated sheet (24), are produced from a perforated sheet and are arranged above the associated perforated sheet (24) and
- with c) a spring device which is arranged between the base rack and the pressure plates (28) and has several springs (plate spring 32), which prestress the pressure plates (28) resiliently against the respectively associated perforated sheet (24).

2. A blood plasma holder according to Claim 1, characterised in that the pressure plates (28) or the base rack, in particular the perforated sheets (24), have spacers which project in the opposite direction to the resilient pressure acting vertically onto the pressure plate (28), and establish a minimal vertical distance of each pressure plate (28) from the associated perforated sheet (24).

3. A blood plasma holder according to Claim 1, characterised in that lateral edges (30) are provided on the pressure plates (28) and/or on the perforated sheets (24) running parallel to the frame parts (20, 22), which lateral edges (30) are preferably connected in one piece with the pressure plate (28) or the perforated sheet (24).

4. A blood plasma mounting according to Claim 1, characterised in that the area of a pressure plate (28) differs by a maximum of 20 % from the area of the associated perforated sheet (24).

5. A blood plasma holder according to Claim 1, characterised in that the spring device has two lateral plate springs (32) for each pressure plate (28), which have a length which corresponds to at least 50 % of the depth dimension of the pressure plate (28).

6. A blood plasma holder according to Claim 1, characterised in that on each pressure plate (28) the springs (plate spring 32) of the spring device only lie laterally and preferably in a linear manner, so that the pressure plate (28) can be pushed upwards out from the springs holding it.

7. A blood plasma holder according to Claim 1, characterised in that two lateral plate springs (32), constructed in an L-shape, are provided in each case per pressure plate (28), which plate springs (32) have a short L-shank which is secured to the perforated sheet (24).

8. A blood plasma holder according to Claim 1, characterised in that the perforated sheets (24) and the pressure plates (28) have holes which form at least 20 % of the area of the respective part.

9. A blood plasma holder according to Claim 1, characterised in that the lateral frame parts (20, 22) are bent from a rod material, in particular round material and are constructed substantially in a rectangular shape.

10. A blood plasma holder according to Claim 1, characterised in that the frame parts (20, 22) are bent inwards at their upper, free ends in the region of an incline (26), whereby a support is achieved for a blood plasma holder stacked thereabove.

## Revendications

1. Support pour poche à plasma pendant la congélation, caractérisée par un bâti empilable métallique comprenant :
- a) un bâti de base, qui comprend deux parties de cadre latérales (20, 22) et au moins trois tôles perforées (24) agencées entre ces parties de cadre latérales et reliées à celles-ci, lesdites tôles perforées étant parallèles les unes aux autres et situées à distance les unes des autres ;
- b) des plaques de compression (28) prévues à raison d'une par tôle perforée (24), qui sont maintenues de façon mobile sur la tôle perforée associée (24), sont réalisées à partir d'une plaque à trous, et sont agencées au-dessus de la tôle perforée associée (24) ; et
- c) un dispositif à ressorts qui est agencé entre le bâti de base et les plaques de compression (28) et comprend plusieurs ressorts (ressorts à lame 32), qui précontraignent les plaques de compression (28) élastiquement contre la tôle perforée associée respective (24).

2. Support pour poche à plasma selon la revendication 1, caractérisé en ce que les plaques de compression (28) ou le bâti de base, en particulier les tôles perforées (24), comportent des éléments d'écartement qui dépassent en direction opposée à la force des ressorts agissant verticalement contre la plaque de compression (28), et qui déterminent une distance verticale minimum entre chaque plaque de compression (28) et la tôle perforée associée (24).

3. Support pour poche à plasma selon la revendication 1, caractérisé en ce que sur les plaques de compression (28) et/ou sur les tôles perforées (24) sont prévues des bordures latérales (30) qui s'étendent parallèlement aux parties latérales du cadre (20, 22), lesdites bordures latérales étant de préférence reliées d'une seule pièce à la plaque de compression (28) ou à la tôle perforée (24).

4. Support pour poche à plasma selon la revendication 1, caractérisé en ce que la surface d'une plaque de compression (28) diffère au maximum de 20% de la surface de la tôle perforée associée (24).

5. Support pour poche à plasma selon la revendication 1, caractérisé en ce que le dispositif à ressorts comporte deux ressorts à lame latéraux (32) pour chaque plaque de compression (28), lesquels ont une longueur qui correspond au moins à 50% de la dimension en profondeur de la plaque de compression (28).

6. Support pour poche à plasma selon la revendication 1, caractérisé en ce que sur chaque plaque de compression (28) les ressorts (ressorts à lame 32) du dispositif à ressorts ne sont appliqués que latéralement et de préférence linéairement, de sorte que la plaque de compression (28) peut être soulevée vers le haut hors des ressorts qui la maintiennent.

7. Support pour poche à plasma selon la revendication 1, caractérisé en ce que qu'il est prévu deux ressorts à lame latéraux (32) réalisés en forme de L pour chaque plaque de compression (28), lesdits ressorts présentant un bras court qui est fixé à la tôle perforée (24).

8. Support pour poche à plasma selon la revendication 1, caractérisé en ce que les tôles perforées (24) et les plaques de compression (28) présentent des trous qui constituent au moins 20% de la surface de la partie correspondante.

9. Support pour poche à plasma selon la revendication 1, caractérisé en ce que les parties de cadre latérales (20, 22) sont réalisées à partir d'un matériau en forme de tige, en particulier un matériau en forme de tige ronde cintrée, et réalisées sensiblement sous forme rectangulaire.

10. Support pour poche à plasma selon la revendication 1, caractérisé en ce que les parties de cadre (20, 22) sont recourbées vers l'intérieur au niveau de leurs extrémités supérieures libres, dans la région d'une partie inclinée (26), grâce à quoi on réalise un appui pour un support pour poche à plasma empilé au-dessus.
